# EUROPÄISCHE PATENTSCHRIFT

(11) **EP 0 555 293 B1**
(45) Veröffentlichungstag und Bekanntmachung des Hinweises auf die Patenterteilung: **21.09.1994**
(21) Anmeldenummer: 91918817.7
(22) Anmeldetag: 31.10.1991
(51) Int. Cl.: A61B 17/08, A61B 17/04

(54) **WUNDVERSCHLUSS**
SURGICAL CLOSURE
FERMETURE POUR PLAIES

(30) Priorität: 31.10.1990 DE 4034705
(43) Veröffentlichungstag der Anmeldung: 18.08.1993
(73) Patentinhaber: NEUMANN, Martin, D-91080 Weiher (DE); Köckerling, Ferdinand, Dr., D-91054 Erlangen (DE)
(72) Erfinder: NEUMANN, Martin, D-91080 Weiher (DE); Köckerling, Ferdinand, Dr., D-91054 Erlangen (DE)
(74) Vertreter: Hufnagel, Walter (DE)
(86) Internationale Anmeldenummer: DE9100849
(87) Internationale Veröffentlichungsnummer: WO9207519

(56) Entgegenhaltungen:
- WO-A-90/09763
- DE-A- 2 418 603
- DE-A- 3 444 782
- DE-C- 111 345
- FR-A- 1 578 758
- US-A- 3 933 158
- US-A- 4 038 989
- US-A- 4 259 959

## Beschreibung

Die vorliegende Erfindung bezieht sich auf einen Wundverschluß gemäß dem Oberbegriff des Anspruches 1.

Ein derartiger Wundverschluß ist beispielsweise aus der DE-PS 34 44 782 bekannt. Dieser Wundverschluß dient insbesondere als passagerer Bauchhöhlenverschluß, vorzugsweise bei der postoperativen Behandlung der Peritonitis.

Die Peritonitis als sekundäre Form, die sich infolge einer Perforation eines Hohlorgans oder als postoperative Komplikation entwickelt, weist auch heute noch eine hohe Letalität auf. Bei steigender Inzidenz stellt sie ein zentrales chirurgisches Problem dar.

Die Bauchhöhle unterliegt einem physiologischen, gerichteten Flüssigkeitsstrom, der seinen Abfluß vor allem über kleine Öffnungen in der peritonealen Zwerchfellunterseite findet. Auf diesem Weg werden die Bakterien über die Lymphbahnen der systemischen Abwehr zugeführt. Die Resorptionsleistung der intraperitonealen Flüssigkeit wird durch die Zwerchfellbeweglichkeit und den intraperitonealen Druck erhöht. Im Rahmen der Peritonitis wird dieser Abfluß durch die pathophysiologische Entstehung von Fibrin und Bakterien verstopft und die Zirkulation durch fibrinbedingte Verwachsungen behindert. Das Abwehrsystem ist gestört und es kommt zu einem Anstieg der Bakterienmassen, bzw. deren Toxinen und Fibrin. Gelingt es nicht die Progredienz der Peritonitis rechtzeitig zu stoppen, kommt eine pathophysiologische Kaskade in Gang, die stetig an Dynamik zunimmt und ab einem gewissen Punkt nicht mehr zu bremsen ist.

Zur Säuberung der Bauchhöhle wird bereits intraoperativ mit physiologischer Kochsalzlösung gespült, bis die Spülflüssigkeit klar bleibt. Mit dieser mechanischen Reinigung sollen Bakterienmassen, Fibrin, devitalisiertes Gewebe, Toxine und auch Blutreste (auch Hämoglobin fördert das Angehen einer Infektion) möglichst vollständig entfernt werden, um zusammen mit der chirurgischen Herdsanierung eine optimale Voraussetzung für die Heilung zu schaffen.

Entscheidend ist es in der postoperativen Phase, in der sich meist das Schicksal des Patienten entscheidet, eine Verschlechterung der Situation so früh wie möglich zu erkennen und gegebenenfalls die Ursache sofort zu beheben (z.B.Korrektur einer Nahtinsuffizienz nach Übernähung eines perforierten Magenulkus) und durch eine effektive Lavage, möglichst vom 1.postoperativen Tag an, für klare Verhältnisse zu sorgen (erneut entstandenes Blut, Fibrin und Bakterien sollen herausgespült werden).

**Bekannt ist** bei der postoperativen Lavage die Strategie des offenen Abdomens bei der Intervallspülung und die Spülbehandlung bei geschlossenen Abdomen.

Dieses sogenannte **offene Abdomen** wird ermöglicht durch den Schienengleitverschluß und durch den Reißverschluß als passageren Bauchhöhlenverschluß mit den **Vorteilen**, daß die wiederholte intraabdominelle Zugänglichkeit gewährleistet ist und sich der Operateur bei jeder Spülung vom Erfolg der Herdsanierung überzeugen und somit den Verlauf der Peritonitis kontrollieren kann. Dabei können postoperative intraabdominelle Verwachsungen gelöst und Fibrinbeläge entfernt werden. Es entfallen die typischen Drainagekomplikationen. (Abdichtung der Drainagen gegenüber der Bauchdecke, Verstopfung oder Verlegung der Drainagen, Infektionsquelle). Eine Relaparotomie ist nicht mehr notwendig.

**Nachteilig** ist dabei, daß direkt nach der Operation nicht gespült werden kann und keine kontinuierliche Spülung möglich ist. Die Intervallspülung ist vor allem dann relativ häufig und zugleich belastend für den Patienten, wenn der Zustand des Patienten kritisch ist. Die Intervallspülung muß sorgfältig vorbereitet werden, sie wird im Operationssaal (bei der Spülung ist das Abdomen offen) und mit Vollnarkose durchgeführt. Auf die Vorteile des Prinzipes der Peritonealdialyse muß verzichtet werden, da die bisherigen passageren Bauchhöhlenverschlüsse das Abdomen nicht dicht verschließen. Der Spüleffekt bleibt eingeschränkt, da ein erwünschter intraabdomineller Druck nicht bestehen bleibt, und die Spülflüssigkeit bevorzugt nur in präformierten Spülstraßen fließt. Außerdem sickert ein Teil der Spülflüssigkeit nach dem provisorischen Verschluß der Bauchdecke in das Bett, was neben einer zusätzlichen Infektionsquelle, uneffektiver Spülung, zusätzlicher Belastung des Patienten einen beträchtlichen Mehraufwand für das Pflegepersonal bedeutet. Patienten mit einem offenen Abdomen gehören derzeit zu den pflegeintensivsten Patienten. Wird ein sog. Reißverschluß oder Schienengleitverschluß als passagerer Bauchhöhlenverschluß eingenäht, kommt ein weiterer Nachteil zum Tragen. Einmal zugeschnitten und eingenäht ist keine Adaption an die Spannungsverhältnisse der Bauchdecke mehr möglich. Durch ödematöse Schwellung innerer Organe im Verlauf einer Peritonitis kann aber die Spannung der Bauchdecke erheblich zunehmen mit der Gefahr, daß die Nähte ausreißen. Auf der anderen Seite müssen die Wundränder später in der Phase der Genesung, in der die inneren Organe abschwellen, wieder schrittweise zur definitiven Bauchdeckennaht zusammengeführt werden. Außerdem ist mit den typischen Reißverschlußkomplikationen zu rechnen (einklemmmen, haken). Es gibt keine besondere Randstruktur zum Einnähen in die Faszie, so daß nur die einzelnen Nähte tragen. Diese sind oft nicht dicht und reißen leicht aus.

Die kontinuierliche Peritoneallavage bei **geschlossenem Abdomen** bietet die **Vorteile**, daß mit einer effektiven Spülbehandlung sofort nach der Operation begonnen werden kann, und damit die Aufgabe der üblichen Redon-Saugdrainage wesentlich effektiver ersetzt werden kann. Diese hat eine schwache Saugleistung, saugt nur da punktuell, wo sie gerade liegt. Außerdem verstopft sie leicht und verleitet dann zu der Annahme, daß der Wundraum bereits leergesaugt ist. Durch das dichte System kann ein intraperitonealer Druck aufgebaut und dosiert werden. Damit kommt die Spülflüssigkeit (eventuell mit Antibiotikazusatz) auch an die kritischen "Wetterecken" des Abdomens. Es entstehen eben nicht nur Spülstraßen wie beim Durchsickern ohne Druck. Es bilden sich weniger fibrinbedingte Vervachsungen, da der Abdomeninhalt "schwimmt" und effektiv Fibrin (unter anderem) ausgespült wird (damit gleichzeitig Ileusprophylaxe).
Peritonealdialyse ist möglich. Damit kann ein Anstieg der Retentionswerte (Kreatinin, Harnstoff, Kalium) bei drohender Niereninsuffizienz denkbar einfach durch Verwendung einer handelsüblichen Dialyseflüssigkeit als Spülflüssigkeit abgefangen werden. Der Patient kann ohne weiteres dialysiert werden, ohne daß er in das aufwendige Programm der Hämodialyse aufgenommen werden muß. Auch fallen die damit verbundenen Nachteile weg, wie sie etwa durch die Manipulation am Blutvolumen entstehen können. Die Spülung kann eine Maschine nach einem gewünschten Schema übernehmen, dadurch ist eine deutliche Entlastung des Pflegepersonals möglich. Durch Kammerzählung der Leukozyten in der Spülflüssigkeit ist eine einfache Erfolgskontrolle der Peritonitis möglich. Eine Auslaufbeurteilung kann denkbar einfach durch Inspektion (Trübung, Fibrin- bzw. Blutbeimengungen) durchgeführt werden. Über die Katheter können Spülwasserproben für Resistenzbestimmung der Bakterien jederzeit entnommen, wie auch andere Substanzen appliziert werden. (Z.B.Elektrolyte,Eiweiß,Heparin ).

**Nachteilig** ist dabei, daß das Abdomen nicht mehr zugänglich ist und dadurch keine direkte Sichtkontrolle mehr besteht, die wichtig ist, wenn sich der Auslauf pathologisch verändert, oder sich der klinische Zustand des Patienten verschlechtert. Abdichtung und Infektionsquelle der Drainagendurchtrittsstellen, sowie Verstopfung oder Verlegung der Drainagen stellen typische Komplikationen dar. Bei einem erneut erforderlichen Zugriff zum Abdomen muß eine Relaparotomie erfolgen.

Trotz der vielversprechenden Ansätze hat sich die Strategie des geschlossenen Abdomens in der Praxis nicht durchsetzen können, da die Nachteile im Vordergrund stehen.

Aufgabe der vorliegenden Erfindung ist es, einen temporären intrakorporalen Wundverschluß der eingangs erwähnten Art so zu verbessern, daß man einen vollkommen druckdichten Verschluß erhält, der aber trotzdem jederzeit beliebig oft zu öffnen ist. Außerdem soll die Möglichkeit zur wiederholbaren, stufenlosen Spannungsregulierung der Bauchdecke bestehen und damit auch des intraperitonealen Druckes, ohne daß die kritische Verbindung zwischen dem Rand des Fixierelementes und der entsprechenden Körpergewebeschicht getrennt werden muß.

Gelöst wird diese Aufgabe durch die im Kennzeichen des Anspruches 1 angegebenen Merkmale.

Mit der vorliegenden Erfindung können die Vorteile der beiden oben beschriebenen Strategien kombiniert werden, so daß neue, erweiterte Therapiemöglichkeiten, beispielsweise in der Bauchchirurgie, zur Verfügung stehen, deren Einsatz nicht nur auf die Behandlung der Peritonitis beschränkt bleibt, sondern beispielsweise auch in der Behandlung der Pankreatitis erfolgversprechend erscheint. Ein passagerer Bauchhöhlenverschluß kommt prinzipiell in Frage, wenn postoperative Komplikationen zu erwarten sind. Der Einsatz ist auch als passagerer Verschluß anderer Körperhöhlen möglich.
Durch integrierte Anschlüsse für Drainageschläuche können gleichzeitig die Vorteile eines kontinuierlichen geschlossenen Spülsystem bzw. der Peritonealdialyse ausgenutzt werden. Eine weitere Verbesserung kann durch einen besonderen Aufbau und durch die Formgebung der Randstruktur erreicht werden, die eine kraftschlüssige und dichte Verbindung zwischen der Grundplatte und der Faszie ermöglicht. Zusätzlich kann ein Fibrinkleber verwendet werden.

Bevorzugte Ausführungsformen der Erfindung ergeben sich aus den Unteransprüchen.

Weitere vorteilhafte Einzelheiten der Erfindung sind nachfolgend anhand der in der Zeichnung veranschaulichten Ausführungsbeispiele näher erläutert. Es zeigen:
- Figur 1: eine Ansicht eines erfindungsgemäßen Wundverschlusses,

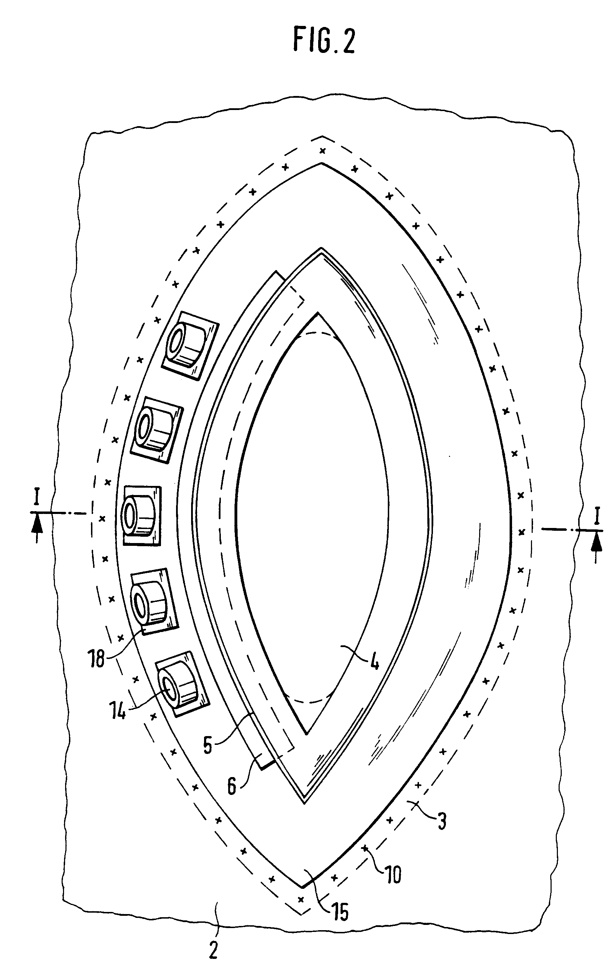

- Figur 2: eine Draufsicht auf den Wundverschluß gemäß Figur 1 im geöffneten Zustand,
- Figur 3: einen Querschnitt, durch den Wundverschluß gemäß Figur 1, gemäß der Schnittlinie I-I in Figur 2,
- Figur 3a: eine Ausschnittsvergrößerung der Einzelheit E der Figur 3,
- Figur 3b und Figur 3c: weitere Verbindungsmöglichkeiten des Fixierelementes mit der Faszie in Form eines Klemmverschlusses,

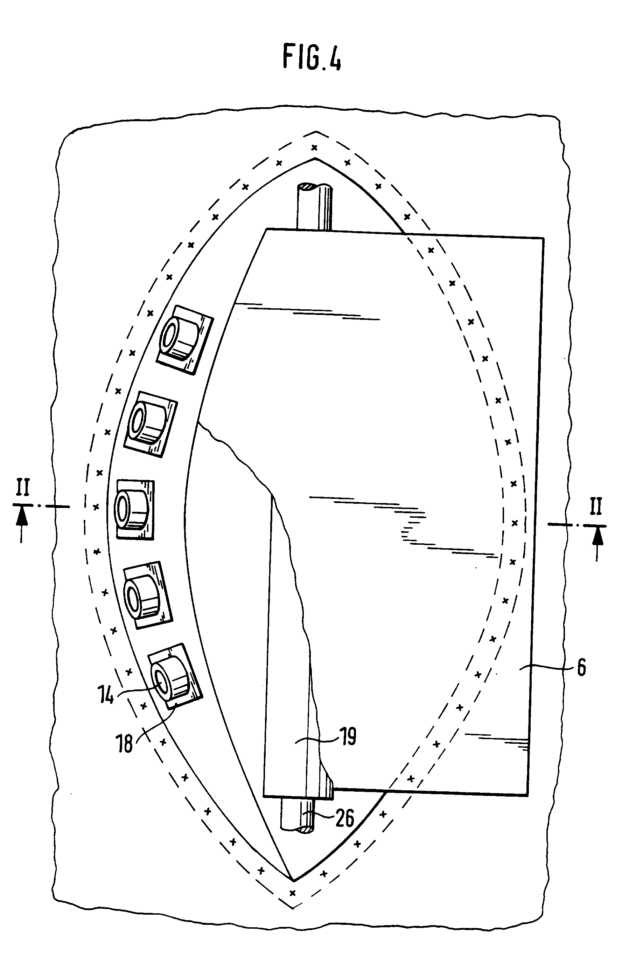

- Figur 4: eine Draufsicht auf den Wundverschluß gemäß Figur 1 im geschlossenen Zustand,
- Figur 5: einen Querschnitt durch den Wundverschluß gemäß Figur 1, gemäß der Schnittlinie II-II in Figur 4 und
- Figur 5a: eine Ausschnittsvergrößerung der Figur 5, betreffend den Stab 26.

Der erfindungsgemäße Wundverschluß kann beispielsweise als passagerer Bauchhöhlenverschluß in die Faszie **1**, das ist die Sehnenplatte der Bauchdecke, an der Unterseite der Bauchwand **2** eingenäht werden.

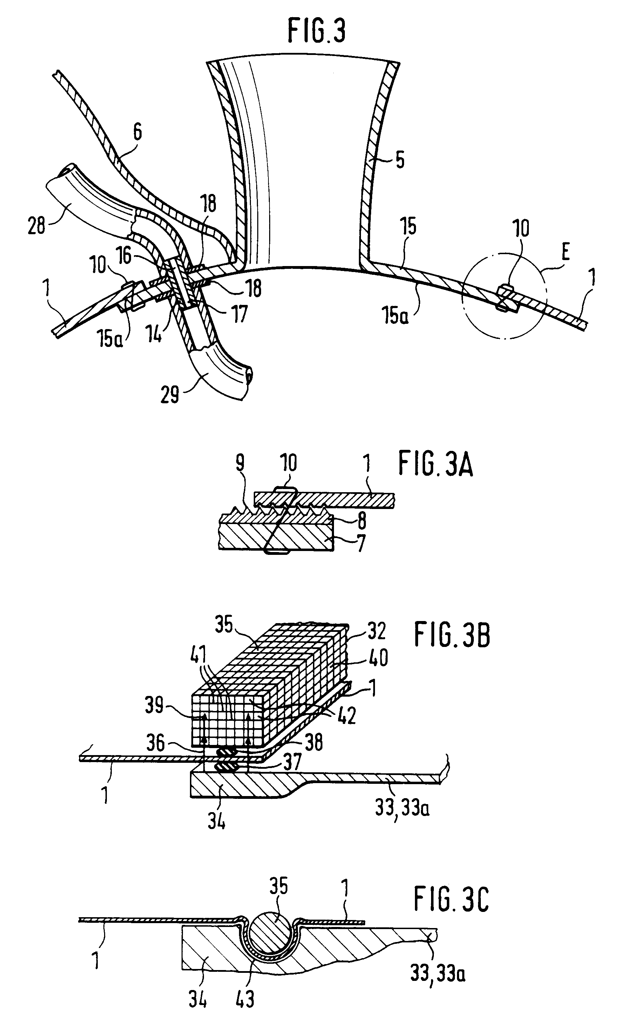

Hierzu wird nach Auswahl der passenden Größe aus einer Mehrzahl von Wundverschlußtypen in Bezug auf die Bauchschnittlänge der Rand **3** eines einzigen, die Wunde allseitig umgebenden Fixierelementes **15**, nachfolgend stets als Grundfläche **15a** bezeichnet, mit seiner oberhalb der Wunde vorzusehenden Öffnung **4** allseitig unter die Faszie **1** geschoben und nach einer möglichen Ausführungsform entsprechend den Figuren **3** und **3a** mit der Faszie **1** vernäht. Dabei kann sich die der Schnittlänge entsprechend gewählte Öffnung **4** an die je nach Spannung der Bauchdecke klaffende Wunde anpassen. Dies wird vor allem dann erreicht, wenn die Öffnung **4** als weit in die Ecken reichender Ausschnitt in Form einer Lanzette oder angenähert einer Ellipse oder eines breiten Spaltes mit gerundeten Stirnseiten ausgebildet ist. Voraussetzung für diese mögliche Adaption der Öffnung **4** ist weiterhin, daß die Grundfläche **15a** aus flexiblem Material besteht, das auch zusätzlich noch elastisch dehnbar sein kann. Die Elastizität sollte aber nicht zu groß sein, um noch eine ausreichende Querspannung in Richtung des Zusammenziehens der Wunde erzeugen zu können.

Die Öffnung **4** ist von einem beispielsweise schlauch- oder trichterförmigen Tubus **5** umgeben, der senkrecht zur Grundfläche **15a** abstehend angeordnet ist und aus flexiblen Material mit gegebenenfalls geringer Elastizität besteht. Unter dem Begriff Tubus ist ein röhrenförmiges Gebilde beliebiger Querschnittsform zu verstehen, das sich von der Öffnung **4** aus, also von der Wunde weg, erstreckt und über seine gesamte Länge eine flüssigkeitsdichte Manschette bildet.

An einer Seite des Tubus **5** ist außerhalb desselben an der Grundfläche **15a** oder am Tubus **5** ein Abdecklappen **6** vorgesehen der nach dem Zusammenlegen, beispielsweise nach dem Zusammenfalten und / oder nach dem Zusammenrollen des Tubus **5** über die Tubusfaltung oder über die Tubusrolle **19** übergeschlagen wird und auf der anderen Seite des Tubus **5** mit der Grundfläche **15a** so befestigbar ist, daß die Überschlagslänge veränderlich ist.

Es ist aber auch eine Ausführungsform denkbar, bei der das Material für die Grundfläche **15a**, für den Tubus **5** und auch für den Abdecklappen **6** hinsichtlich der Elastizität so ausgewählt ist, daß eine plötzlich auftretende intraabdominelle Druckerhöhung, wie etwa ein Hustenstoß, zumindest teilweise abgefangen werden kann. Dadurch können die auf die Verbindung zwischen der Grundfläche **15a** und Faszie **1** auftretenden Kräfte jedenfalls reduziert werden.

Um eine flächig greifende, kraftschlüssige und gewebeverträgliche Verbindung zwischen der Grundfläche **15a** und der Faszie **1** zu ermöglichen, muß der Rand 3 des Fixierelementes die Faszie **1** allseitig ausreichend weit unterlappen bzw. untergreifen, aus biokompatiblem oder gewebeverträglichem Material bestehen und eine geeignete Struktur aufweisen. Als biokompatibles Material eignet sich insbesondere ein unter dem Handelsnamen "Gore-tex" bekannter Textilstoff, wie er bereits in der Gefäßchirurgie verwendet wird. Derartige Blutgefäßprothesen können dicht eingenäht werden und widerstehen Blutdruckspitzen bis über 200 mmHg systolisch.

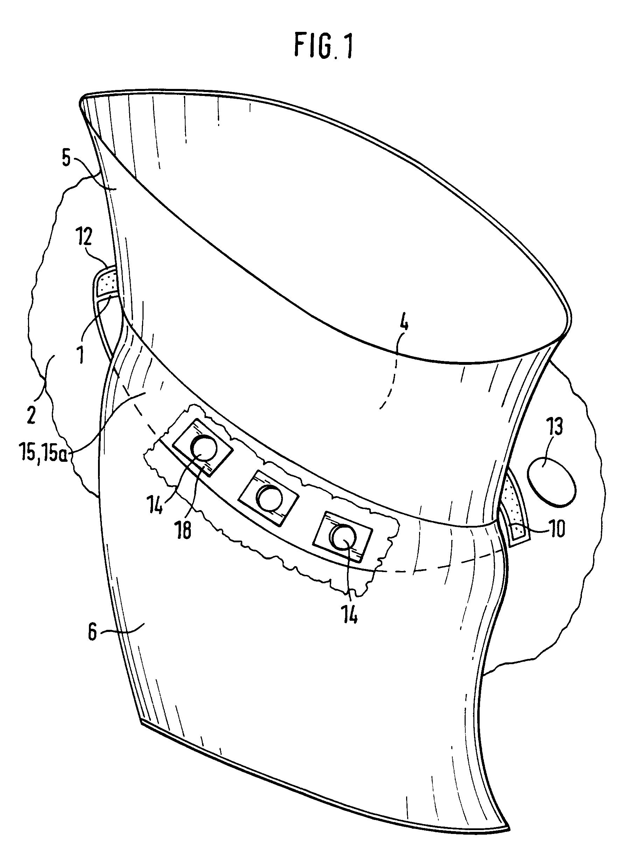

Im Rahmen der vorliegenden Erfindung bestehen mehrere Möglichkeiten in der Ausbildung des Randes **3**. Genügt eine einfache Ausführung nicht den Anforderungen einer stabilen und dichten Verbindung zwischen der Grundfläche **15a** und der Faszie **1**, auch nicht mit der Unterstützung eines Klebstoffes, vorzugsweise eines Fibrinklebers, dann kann der Rand **3**, wie in Figur **3a** dargestellt, ausgebildet sein. Eine ausreichend steife untere Schicht **7** verteilt den Druck, der durch die Naht bzw. durch mehrere Nähte **10** erzeugt wird, großflächig gegen die Faszie **1** und bietet gleichzeitig eine abstützende Schicht für die feinen Spitzen oder Zähne **9**, die in der Faszie **1** flächig greifen. Die Spitzen oder Zähne **9** durchbrechen eine weiche Dichtungsschicht **8**, die oberhalb der unteren Schicht **7** gelegen ist. Die Naht **10** faßt im einfachsten Falle nur die Faszie **1**, kann aber auch nach Nahttechniken, wie bei einem Platzbauch, alle Schichten der Bauchwand **2** erfassen und sich über eine druckverteilende Scheibe **13** (Figur **1**) auf der Haut **12** abstützen. Weiterhin ist es denkbar, daß zur Erstellung einer dichten Naht an der Faszie **1** ein Näh- bzw. Klammergerät zum Einsatz kommt. Auch kann die Verbindung zusätzlich mit einem Fibrinkleber unterstützt und abgedichtet werden. In diesem Zusammenhang ist auch der Einsatz von Silikon als zusätzliches Abdichtungsmaterial denkbar.

Besonders vorteilhaft ist es bei der Herstellung der Naht **10** zwischen der Faszie **1** und der Grundfläche **15a**, daß man durch die zentrale öffnung **4** einen ungehinderten Zugang zu der Operationswunde hat, da der geöffnete Tubus **5** auf Grund seiner Flexibilität flach zusammenfällt bzw. flach zusammengelegt werden kann. Die Nadelführung auf der intraperitonealen Seite kann sichtbar kontrolliert werden. Gegebenenfalls kann bzw. können Bauchhöhleninhalt (e), beispielsweise Darmabschnitte, aus dem Weg der intraperitonealen Nadelführung gedrückt werden. Neben dem Rand **3** sind bevorzugt auf einer Seite des Fixierelementes **15** mehrere Drainageanschlüsse **14** in die Grundfläche **15a** integriert oder nachträglich dort anbringbar, die mit einem Schraubgewinde, sowohl für den bzw. die nach außen führenden oberen Stutzen **16** , als auch für den bzw. die nach innen führenden unteren Stutzen **17** ausgestattet sind. Sind keine Drainagen im Gebrauch, werden die oberen Stutzen **16** mit Schraubstöpseln verschlossen. Die Drainagedurchtritte sind in einer bevorzugten Ausführungsform in zwei Verstärkungsplatten **18** eingearbeitet, die die Grundfläche **15a** von oben und unten flächig und druckdicht einfassen. Wird eine oder werden mehrere Drainagen gebraucht, können sie bei geöffneten Tubus **5** in der Bauchhöhle plaziert und an den unteren Stutzen **17** angeschraubt werden. Die extrakorporale Drainage **28** kann als Fortsetzung auch bei geschlossenem Tubus **5** an den oberen Stutzen **16** angeschraubt werden. Da hierbei die Probleme der Dichtigkeit und einer Infektionsquelle an einer undichten Drainagedurchtrittsstelle entfallen, wie sie zum Teil bei der seperaten Katheterdurchführung durch die Bauchdecke auftreten, kann man sich einfacher zur Benutzung mehrerer Drainagen über die integrierten Drainageanschlüsse **14** entscheiden und das System grundsätzlich auch für größerlumige Drainagen auslegen. Durch die einfache intraabdominelle Zugänglichkeit hat man hier die Möglichkeit, auch bei Funktionsverlust der intrakorporalen Drainagen **29** die intraabdominelle Ursache durch die Öffnung **4** zu beheben.

Am Innenrand der Grundfläche **15a** ansetzend, erhebt sich der nach oben offene Tubus **5**. Dieser ist mit der Grundfläche **15a** fest und druckdicht verbunden und kann die zentrale Öffnung **4** und damit die Bauchhöhle druckdicht abschließen.

Gemäß einem bevorzugten Ausführungsbeispiel wird der Tubus **5** durch einen Einrollvorgang über einen beispielsweise zweigeteilten Stab **26** verschlossen.

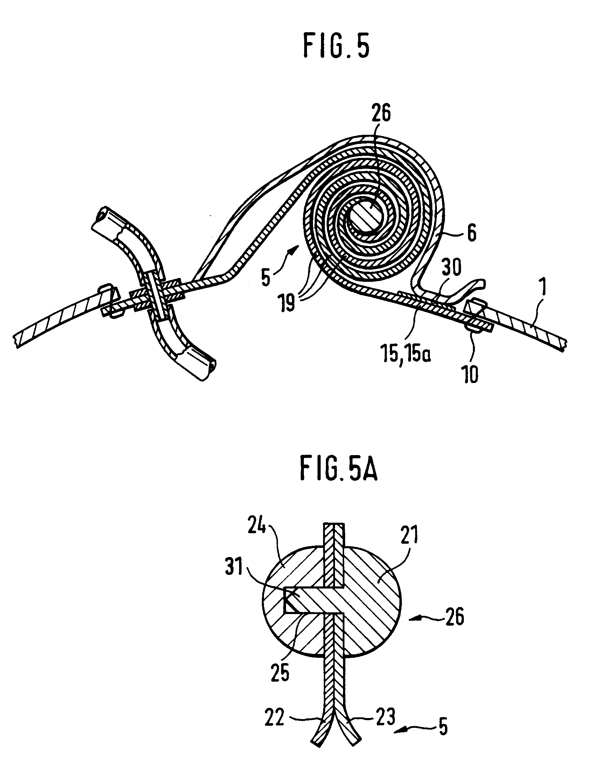

Figur **5a** zeigt, daß die eine Stabhälfte **21** mit spitzen Stiften **31** besetzt ist, die die beiden oben zusammengelegten Wände **22**, **23** des Tubus **5** durchstechen, wobei die zusammengelegten Tubuswände **22**, **23** vorher gefaltet werden können. Die zweite Stabhälfte **24** ist über entsprechende Aufnahmen oder Löcher **25** auf die Stifte **31** der ersten Stabhälfte **21** gedrückt. Auf diese Weise fixiert, kann der zusammengeführte Tubus **5** über den als Rundstab ausgebildeten Stab **26** eingerollt und zu einer Tubusrolle **19** geformt werden. Je nachdem, wie weit gerollt wird und wie das Längsschnittprofil des Stabes **26** gestaltet ist, können unterschiedliche Teilspannungen auf die Wundränder erzeugt werden. Der eingerollte Tubus **19** wird nun abschließend mit einem Abdecklappen **6** fixiert. Dieser Abdecklappen **6** ist auf der Seite der Drainageanschlüsse **14** mit der Grundfläche **15a** fest verbunden. Er greift über den eingerollten Tubus **19** und findet auf der gegenüberliegenden Seite halt. Vorzugsweise wird zu diesem Zweck ein Haftverschluß, wie ein Klettverschluß **30**, verwendet.

Bei einer anderen Ausführungsform kann der Stab **26** ausschließlich als Rollhilfe verwendet werden und nach dem Einrollvorgang herausgezogen werden. Die Enden der Tubusrolle **19** können auch einwärts gefaltet und so von dem übergreifenden Abdecklappen **6** fixiert werden. In besonderen Fällen kann auch eine Art Bauchgurt als zusätzliche Sicherung sinnvoll sein.

Nach einer weiteren Ausführungsform, wie in Figur **3b** dargestellt, kann der dichte Verschluß zwischen der Faszie **1** und der Grundfläche **33a** des Fixierelementes **33** als Klemmverschluß **32** wie folgt ausgebildet sein.

Um eine flächig greifende, kraftschlüssige, gegebenenfalls kombinierte kraft- und formschlüssige, sowie dichte Verbindung zwischen der Grundfläche **33a** des Fixierelementes **33** und der Faszie **1** zu ermöglichen, muß der innere Rand **34** des Fixierelementes **33** die Faszie **1** allseitig ausreichend weit untergreifen bzw. unterlappen, so daß von oben auf den inneren Rand **34** gedrückte Klemmstücke **35** die Faszie **1** ausreichend haltbar und dicht klemmen können.

Wie die Figur **3b** zeigt, besitzt der innere Rand **34** des Klemmverschlusses **32** vorzugsweise eine Breite von etwa **2** cm und er ist mit Widerhaken **36** besetzt, die vorzugsweise ein bis zwei cm lang sind. Gegen ein von oben aufgesetztes Klemmstück **35** gedrückt, durchstechen diese Widerhaken **36** von unten die Faszie **1** und eventuell auch einen Teil der sich nach oben anschließenden Muskelschicht. In den Klemmstücken **35**, die bevorzugt als Gitterblock **40** aufgebaut sind, können die Widerhaken **36** in die einzelnen Öffnungen des Gitterblockes **40**, je nach Druck unterschiedlich weit, in die entsprechenden horizontalen Schichten bzw. Gitterebenen **41** des Gitterblockes **40** einrasten. Zusätzliche innere und äußere Dichtungen **37**, **38** dichten den Klemmverschluß **32** flüssigkeitsdicht ab. Es ist auf einfache Weise möglich, die beiden komplementären Klemmteile **35**, **36** gleichzeitig mit dem Daumen und dem Zeigefinger einer Hand zusammenzudrücken, da durch die zentrale Öffnung **4** der Zeigefinger um den zusammengelegten Tubus **5** herum unter die Grundfläche **33a** greifen kann. Die einzelnen Klemmstücke **35** mit einem geeigneten Schneidwerkzeug in Form und Länge passend geschnitten, dichten den Wundrand umlaufend ab.

Bei der Verwendung von Gitterblöcken **40** als Klemmstücke **35** bestehen diese aus ein oder mehreren Gitterebenen **41**, die zur Herstellung einer festen Rastverbindung mit Öffnungen **42** versehen sind, die zu den Zacken **39** der Widerhaken **36** korrespondieren.

Um eine sichere Abdichtung des Klemmverschlusses gemäß Figur **3b** sicherzustellen, sind im untergreifenden Randbereich **34** des Fixierelementes **33** zwischen diesem und der Faszie **1** einerseits und zwischen der Faszie **1** und den Klemmstücken **35** andererseits die Wundöffnung **4** vollständig umfassende Dichtungselemente **37**, **38** vorgesehen.

Wichtig ist, daß bei Verwendung von mehreren Gitterblöcken **40** längs der Wundöffnung **4** diese voneinander so weit beabstandet sind, daß sie längs der Wundöffnung **4** seitlich gegeneinander verschiebbar sind.

Nach einer anderen Ausführungsvariante können die Klemmstücke **35** aus elastisch nachgiebigem Vollmaterial bestehen, beispielsweise aus einem gummielastischen Material oder aus gummielastischen Kunststoffen, in die die Widerhaken **36** eindrückbar sind, um eine ausreichend kraftschlüssige Verbindung herzustellen.

Die Lösung der Gitterblöcke **40** oder allgemein der Klemmstücke **35** vom inneren Rand der Grundfläche **33a** des Fixierelementes **33** erfolgt zweckmäßig über an sich bekannte Hebelwerkzeuge.

Gemäß dem Ausführungsbeispiel nach Figur **3c**, weist der innere Rand **34** der Grundfläche **33a** des Fixierelementes **33** eine die Wundöffnung 4 vollständig umgebende Ausnehmung **43** auf, die sich oberflächlich in Richtung zur Faszie **1** hin im Querschnitt verjüngt, so daß eine oder mehrere Klemmstücke **35** in diese Ausnehmung **43** unter Zwischenlage der Faszie **1**, gegebenenfalls in Verbindung mit weiteren Körpergewebestrukturen, als Dichtungselement verrastbar sind. Bevorzugt besitzen die Klemmstücke **35** einen kreisrunden Querschnitt, so daß sie in die ebenfalls wenigstens annähernd kreisrunde Ausnehmung **43** einschnappbar und aufgrund der elastischen Ausbildung des Fixierelementes **33** in dieser Lage kraft- und formschlüssig fixiert sind. Dieser atraumatische Klemmverschluß zeichnet sich dadurch aus, daß ein bevorzugt geringgradig flexibler, schneidbare Stab, vorzugsweise Rundstab, in die erwähnte Ausnehmung **43** gedrückt werden kann, und daß bei dieser Klemmverbindung sichergestellt ist, daß die dazwischenliegende Faszie **1** nicht verletzt wird.

In Ergänzung der bisherigen Ausführungen kann es auch zweckmäßig sein, an der Grundfläche **15a** bzw. **33a** des Fixierelementes **15** bzw. **33** Markierungen, beispielsweise in Form von Zahlen, Buchstaben, Symbolen oder dergleichen anzubringen.

Günstig ist es auch, wenn der gesamte Wundverschluß, oder zumindest diejenigen Teile hiervon, die direkt mit dem Körpergewebe in Berührung kommen oder kommen können, aus einem biokompatiblen Material bestehen, wie beispielsweise der früher erwähnte, unter dem Handelsnamen "Gore-tex" bekannte Textilstoff.

Auch wenn, wie insbesondere aus den Figuren **3** und **5** ersichtlich, der Tubus **5** und das Fixierelement **15** bevorzugt einteilig ausgebildet sind, liegt es im Rahmen einer möglichen Abwandlung der Erfindung, den Tubus **5** und das Fixierelement **15** als gesonderte Bauteile auszugestalten und diese anschließend miteinader dicht zu verbinden.

Nach einer bevorzugten Ausführungsform besteht das Fixierelement **15** aus einem einzigen Bauteil. Dies schließt jedoch nicht aus, das Fixierelement **15** aus mehreren Fixierelementteilen herzustellen und diese dicht miteinander zu verbinden.

Als Materialien für den Tubus **5** kommen insbesondere nachgiebige Kunststoffolien oder textile Kunststoffe, die oberflächlich mit Kunststoffen oder mit anderen dichtenden und / oder gewebeverträglichen Materialien versehen sein können in Betracht.

Weitere Vorteile des erfindungsgemäßen Wundverschlusses sind in der Anwendung der Laparoskopie zu sehen. Zu diesem Zweck können beispielsweise Adapterstücke an den Drainageanschlüssen **14** angebracht werden, durch die hindurch laparoskopische Untersuchungen und / oder Eingriffe vorgenommen werden können.

Schließlich ist es auch von Vorteil, daß der erfindungsgemäße Wundverschluß die Möglichkeit für unterschiedliche Spül- bzw. Spül- Saugschemata eröffnet. Auch die postoperative kontinuierliche offene dorsoventrale Bauchspülung läßt sich mit dem passageren Bauchdeckenverschluß gemäß der Erfindung leicht realisieren. Die umständliche, nachteilige Palisadenstabilisierung der Wundöffnung fällt hierbei weg. Die Drainageanschlüsse **14** können als Spülzulauf und eine in der zentralen Tubusöffnung **4** plazierte und mit dem Tubus **5** abgedichtete, beispielsweise mit einer Schlauchklemme oder einem Schnellverbinder, großlumige Drainage als Saugrohr verwendet werden. Außerdem kann über einen Drainageanschluß **14** als permanenten Zugang eine Sonde zur Messung und laufenden Kontrolle des intraperitonealen Druckes angeschlossen werden.

Schließlich ist durch den Klemmverschluß gemäß der Erfindung eine schnellere Implantation als durch das zeitaufwendige Einnähen möglich.

## Patentansprüche

1. Wundverschluß, der wiederholt geöffnet und geschlossen werden kann, insbesondere Bauchdecken-Wundverschluß, bestehend aus mit dem der Wunde benachbarten Körpergewebe fest, jedoch lösbar verbindbaren flexiblen tuch- oder plattenförmigen Fixierelementen und einem die freien Endbereiche derselben oberhalb der Wunde schließenden Verschluß, der jederzeit geöffnet und geschlossen werden kann, dadurch gekennzeichnet, daß nur ein einziges die Wunde ringsum umfassendes Fixierelement (15) vorgesehen ist, das eine oberhalb der Wunde anzubringende Öffnung (4) aufweist, und aus einem die Öffnung (4) umgebenden, sich von der Öffnung (4) von der Wunde weg erstreckenden Tubus (5) aus flexiblem Material besteht und der Tubus (5) selbst als Verschluß dient oder mit einem solchen versehen ist.

2. Wundverschluß nach Anspruch 1, dadurch gekennzeichnet, daß die Öffnung (4) lanzett- oder ellipsenförmig ausgebildet ist.

3. Wundverschluß nach Anspruch 1 oder 2, dadurch gekennzeichnet, daß der Tubus (5) am Fixierelement (15) druckdicht angeordnet ist.

4. Wundverschluß nach einem der Ansprüche 1 bis 3, dadurch gekennzeichnet, daß das Fixierelement (15) in dem den Tubus (5) außen umgebenden Bereich wenigstens einen Drainageschlauchanschluß (14) aufweist oder das Fixierelement (15) mit wenigstens einem solchen Anschluß bestückbar ist.

5. Wundverschluß nach einem der Ansprüche 1 bis 4, dadurch gekennzeichnet, daß auf einer Seite außen neben dem Tubus (5) ein Abdecklappen (6) angebracht ist, der den zusammengelegten Tubus (5) überdecken kann und der auf der seiner Befestigungsseite gegenüberliegenden Seite derart lösbar befestigbar ist, daß seine Überdeckungslänge einstellbar ist.

6. Wundverschluß nach Anspruch 5, dadurch gekennzeichnet, daß zur Befestigung des freien Endes des Abdecklappens (6) ein Haftverschluß (30) vorgesehen ist.

7. Wundverschluß nach einem der Ansprüche 1 bis 6, dadurch gekennzeichnet, daß der Tubus (5) zusammenfaltbar ist.

8. Wundverschluß nach einem der Ansprüche 1 bis 6, dadurch gekennzeichnet, daß der Tubus (5) zusammenrollbar ist.

9. Wundverschluß nach einem der Ansprüche 1 bis 8, dadurch gekennzeichnet, daß der Tubus (5) am freien Rand beginnend, über einen Stab (26) zusammenrollbar ist.

10. Wundverschluß nach Anspruch 9, dadurch gekennzeichnet, daß der Tubus (5) im Bereich seines freien Randes Fixiermittel zum Befestigen oder / und Heften des Stabes (26) aufweist.

11. Wundverschluß nach Anspruch 9 oder 10, dadurch gekennzeichnet, daß der Stab (26) entlang seiner Längsachse unterschiedliche Durchmessergrößen besitzt.

12. Wundverschluß nach einem der Ansprüche 9 bis 11, dadurch gekennzeichnet, daß bei zusammengerolltem Tubus (5) im Bereich der gebildeten Tubusrolle (19) eine Fixiervorrichtung (30) zum Halten der Tubusrolle (19) vorgesehen ist.

13. Wundverschluß nach Anspruch 12, dadurch gekennzeichnet, daß zur Fixierung ein Haftverschluß (30) vorgesehen ist.

14. Wundverschluß nach einem der Ansprüche 1 bis 13, dadurch gekennzeichnet, daß er zumindest in dem unmittelbar mit Körpergewebe direkt in Berührung kommenden Bereich aus biokompatiblem Material besteht.

15. Wundverschluß nach einem der Ansprüche 1 bis 14, dadurch gekennzeichnet, daß das Fixierelement (15) eines oder mehrere Markierungszeichen aufweist.

16. Wundverschluß nach einem der Ansprüche 4 bis 15, dadurch gekennzeichnet, daß Drainageschlauchanschlüsse (14) mit unterschiedlichem Durchmesser vorgesehen oder anbringbar sind.

17. Wundverschluß nach einem der Ansprüche 4 bis 15, dadurch gekennzeichnet, daß die Drainageschlauchanschlüsse (14) gegen Verdrehen gesichert angeordnet sind.

18. Wundverschluß nach einem der Ansprüche 1 bis 17, dadurch gekennzeichnet, daß zum Zusammenrollen ein längsgeteilter Stab (26) vorgesehen ist, dessen eine Stabhälfte (21) nach innen abstehende Stifte (31) aufweist, die in Bohrungen (25) der anderen Stabhälfte (24) einsetzbar bzw. einklemmbar sind und die die Trennwände (22, 23) des Tubus(5) durchstoßen können.

19. Wundverschluß nach Anspruch 1, dadurch gekennzeichnet, daß die Verbindung der Faszie (1) über einen Klemmverschluß (32) hergestellt ist (Figuren 3b und 3c).

20. Wundverschluß nach Anspruch 19, dadurch gekennzeichnet, daß der die Faszie (1) untergreifende Randbereich (34) der Grundfläche (33a) des Fixierelementes (33) mit zur Faszie (1) ausgerichteten Widerhaken (36) versehen ist, die mit auf die Faszie (1) von oben aufbringbaren Klemmstücken (35) kraft- und / oder formschlüssig verbindbar sind.

21. Wundverschluß nach Anspruch 19 oder 20, dadurch gekennzeichnet, daß die Klemmstücke (35) als Gitterblock (40) ausgebildet sind.

22. Wundverschluß nach Anspruch 21, dadurch gekennzeichnet, daß die Gitterblöcke (40) aus einer oder mehreren Gitterebenen (41) bestehen, die zwecks einer Rastverbindung mit den Zacken (39) der Widerhaken korrespondierende Öffnungen (42) aufweisen.

23. Wundverschluß nach einem der Ansprüche 19 bis 22, dadurch gekennzeichnet, daß im untergreifenden Randbereich (34) des Fixierelementes (33) zwischen diesem und der Faszie (1) einerseits und zwischen der Faszie (1) und den Klemmstücken (35) andererseits die Wundöffnung (4) vollständig umfassende Dichtungselemente (37, 38) vorgesehen sind.

24. Wundverschluß nach einem der Ansprüche 20 bis 23, dadurch gekennzeichnet, daß die Klemmstücke (35) voneinander beabstandet sind, damit sie längs der Wundöffnung (4) seitlich gegeneinander verschiebbar sind.

25. Wundverschluß nach Anspruch 20, dadurch gekennzeichnet, daß die Klemmstücke (35) aus elastisch nachgiebigem Vollmaterial bestehen, in die die Widerhaken (36) eindrückbar sind.

26. Wundverschluß nach Anspruch 19, dadurch gekennzeichnet, daß der innere Rand (34) der Grundfläche (33a) des Fixierelementes (33) eine die Wundöffnung (4) vollständig umgebende Ausnehmung (43) aufweist, die sich oberflächlich in Richtung Faszie (1) hin im Querschnitt verjüngt, so daß ein oder mehrere Klemmstücke (35) in dieser Ausnehmung (43) unter Zwischenlage der Faszie (1), gegebenenfalls in Verbindung mit weiteren Körpergewebeschichten, als Dichtungs- und / oder Halteelement verrastbar sind (Figur 3c).

## Claims

1. A surgical closure which my be opened and closed repeatedly, in particular a closure for an incision in the abdominal wall, and consists of flexible fixing elements in the form of cloth or plates, which may be connected firmly but detachably to the body tissue adjacent to the incision, and a closure which closes the free end regions of the said fixing elements above the incision and my be opened and closed at any time, characterized in that only one single fixing element (15) is provided, which encloses the incision all round and exhibits an opening (4) to be fitted above the incision, and consists of a tube (5) of flexible material, which surrounds the opening (4) and extends away from the wall from the opening (4), and the tube (5) itself acts as closure or is provided with one.

2. A surgical closure as in Claim 1, characterized in that the opening (4) is made lanceolate or elliptical.

3. A surgical closure as in Claim 1 or 2, characterized in that the tube (5) is arranged on the fixing element (15) to be pressuretight.

4. A surgical closure as in one of the Claims 1 to 3, characterized in that the fixing element (15) in the region surrounding the outside of the tube (5) exhibits at least one terminal (14) for a drainage tube or the fixing element (15) is armed with at least one such terminal.

5. A surgical closure as in one of the Claims 1 to 4, characterized in that a cover flap (6) is fitted next the outside of the tube (5) on one side and can cover over the collapsed tube and at the side opposite the side where it is fixed can be fastened detachably in such a way that its length of cover is adjustable.

6. A surgical closure as in Claim 5, characterized in that for fastening the free end of the cover flap (6) a cling closure (30) is provided.

7. A surgical closure as in one of the Claims 1 to 6, characterized in that the tube (5) may be folded together.

8. A surgical closure as in one of the Claims 1 to 6, characterized in that the tube (5) may be rolled together.

9. A surgical closure as in one of the Claims 1 to 8, characterized in that starting at the free edge the tube (5) may be folded together over a rod (26).

10. A surgical closure as in Claim 9, characterized in that in the region of its free edge the tube (5) exhibits fixing means of fastening and/or tacking the rod (26).

11. A surgical closure as in Claim 9 or 10, characterized in that the rod (26) has different diameters along its longitudinal axis.

12. A surgical closure as in one of the Claims 9 to 11, characterized in that with the tube (5) rolled together a fixing device (30) is provided in the region of the rolled tube (19) so formed, for holding the rolled tube (19).

13. A surgical closure as in Claim 12, characterized in that a cling closure (30) is provided for the fixing.

14. A surgical closure as in one of the Claims 1 to 13, characterized in that at least in the region which comes directly into direct contact with body tissue it consists of biocompatible material.

15. A surgical closure as in one of the Claims 1 to 14, characterized in that the fixing element (15) exhibits one or more marking signs.

16. A surgical closure as in one of the Claims 4 to 15, characterized in that drainage tube terminals (14) of different diameters are provided or may be fitted.

17. A surgical closure as in one of the Claims 4 to 15, characterized in that the drainage tube terminals (14) are arranged so as to be secured against twisting.

18. A surgical closure as in one of the Claims 1 to 17, characterized in that for rolling it together a rod (26) divided lengthwise is provided, one half-rod (21) of which exhibits pins (31) which project inwards and may be inserted or jammed in holes (25) drilled in the other half-rod (24) and pierce the dividing walls (22, 23) of the tube (5).

19. A surgical closure as in Claim 1, characterized in that the connection to the fascia (1) is produced (Figures 3b and 3c) via a clamp closure (32).

20. A surgical closure as in Claim 19, characterized in that that region (34) which engages under the fascia (1), of the edge of the base (33a) of the fixing element (33), is provided with barbs (36) which are aligned at the fascia (1) and may be connected positively or frictionally to clamping-pieces (35) which may be applied to the fascia (1) from above.

21. A surgical closure as in Claim 19 or 20, characterized in that the clamping-pieces (35) are made as a grid block (40).

22. A surgical closure as in Claim 21, characterized in that the grid blocks (40) consist of one or more grid planes (41) which for the purpose of a snap-connection to the teeth (39) of the barbs exhibit corresponding openings (42).

23. A surgical closure as in one of the Claims 19 to 22, characterized in that in the region (34) engaging under the fascia (1), of the edge of the fixing element (33), between the latter and the fascia (1) on the one hand and between the fascia (1) and the clamping-pieces (35) on the other, sealing elements (37, 38) are provided, which completely enclose the incised opening (4).

24. A surgical closure as in one of the Claims 20 to 23, characterized in that the clamping-pieces (35) are spaced apart so that they may be shifted mutually sideways along the incised opening (4).

25. A surgical closure as in Claim 20, characterized in that the clamping-pieces (35) consist of elastically yielding solid material into which the barbs (36) may be forced.

26. A surgical closure as in Claim 19, characterized in that the inner edge (34) of the base (33a) of the fixing element (33) exhibits a recess (43) which completely surrounds the incised opening (4) and at the surface narrows in cross-section in the direction towards the fascia (1) so that one or more clamping-pieces (35) may be snapped as sealing and/or retainer elements into this recess (43) with the interposition of the fascia (1), if necessary in combination with other layers of body tissue (Figure 3c).

## Revendications

1. Fermeture pour plaies, pouvant être ouvert et fermé à répétition, notamment obturateur de blessures de la paroi abdominale, comportant des éléments de fixation flexibles en forme de tissu ou de feuille, pouvant être fixés de manière amovible, aux tissus organiques proches de la blessure, et dont les extrémités libres, au dessus de la blessure, sont fermées par un obturateur qui peut être ouvert ou fermé à tout moment, caractérisée en ce qu'il est prévu un unique élément de fixation (15) disposé tout le long de la périphérie de la blessure, cet élément comportant une ouverture (4) au-dessus de la blessure ainsi qu'un tube (5) en matériau flexible partant de l'ouverture (4) vers l'extérieur de la blessure et jouant lui-même le rôle d'obturateur ou étant équipé d'un obturateur spécifique.

2. Fermeture pour plaies selon la revendication 1, caractérisée en ce que l'ouverture (4) a une forme en lanzett ou elliptique.

3. Fermeture pour plaies selon la revendication 1 ou 2, caractérisée en ce que le tube (5) est monté de manière étanche sur l'élément de fixation (15).

4. Fermeture pour plaies selon une des revendications 1 à 3, caractérisée en ce que l'élément de fixation (15) est équipé, ou peut être équipé à la périphérie externe du tube (5), d'au moins un raccord pour tuyau de drainage (14) ou l'élément de fixation (15) est susceptible d'être constitué au moins par un tel obturateur.

5. Fermeture pour plaies selon une des revendications 1 à 4, caractérisée en ce que sur un côté du tube (5) et à l'extérieur, est monté un volet le couverture (6) qui peut recouvrir le tube (5) rassemblé sur lui-même et qui peut être du côté opposé à celui de son montage, fixé de manière amovible, sa longueur de recouvrement étant réglable.

6. Fermeture pour plaies selon la revendication 5, caractérisée en ce que pour fixer l'extrémité libre du volet de couverture (6) une fermeture (30) par adhérence est prévue.

7. Fermeture pour plaies selon une des revendications 1 à 5, caractérisée en ce que le tube (5) peut être plié sur lui-même.

8. Fermeture pour plaies selon une des revendications 1 à 6, caractérisée en ce que le tube (5) peut être roulé sur lui-même.

9. Fermeture pour plaies selon une des revendications 1 à 8, caractérisée en ce que le tube (5) peut, à partir de son bord libre être enroulé sur une tige (26).

10. Fermeture pour plaies selon la revendication 9, caractérisée en ce que le tube (5) est équipé, le long de son bord libre, de moyens permettant de le fixer et/ou de le faire adhérer à la tige (26).

11. Fermeture pour plaies selon la revendication 9 ou 10, caractérisée en ce que la tige (26) présente, le long de son axe, des diamètres différents.

12. Fermeture pour plaies selon une des revendications 9 à 11, caractérisée en ce qu'il est prévu un dispositif (30) de fixation du rouleau (19) formé par le tube (5) enroulé sur lui-même dans la zone du rouleau du tube (19) ainsi réalisé.

13. Fermeture pour plaies selon la revendication 12, caractérisée en ce que le dispositif de fixation est une fermeture par adhérence (30).

14. Fermeture pour plaies selon une des revendications 1 à 13, caractérisée en ce qu'il est composé, au moins dans sa partie venant directement au contact des tissus organiques, d'un matériau biocompatible.

15. Fermeture pour plaies selon une des revendications 1 à 14, caractérisée en ce que l'élément de fixation (15) porte un ou plusieurs signes de marquage.

16. Fermeture pour plaies selon une des revendications 4 à 15, caractérisée en ce qu'il comporte ou qu'il peut recevoir des raccords pour tubes de drainage (14) de différents diamètres.

17. Fermeture pour plaies selon une des revendications 4 à 15, caractérisée en ce que les raccords (14) sont montés de manière à ne pas pouvoir tourner.

18. Fermeture pour plaies selon une des revendications 1 à 17, caractérisée en ce qu'il est prévu, pour l'enroulage, une tige (26) divisée longitudinalement, dont une moitié (21) porte des pointes (31), dépassant vers l'intérieur que l'on peut engager, c'est-à-dire faire enserrer par des trous (25) de l'autre moitié et qui peuvent traverser les parois (22, 23) du tube (5)

19. Fermeture pour plaies selon la revendication 1, caractérisée en ce que la liaison de l'aponévrose (1) est assurée par une fermeture à pincement (32) (figures 3b et 3c).

20. Fermeture pour plaies selon la revendication 19, caractérisée en ce que la portée (33a) de l'élément de fixation (33) en prise avec le bord (34) de l'aponévrose (1) est équipée de crochets (36) dirigés vers l'aponévrose et constituant les contreparties de pièces de pincement (35) qui peuvent être déposées par l'extérieur sur l'aponévrose et auxquelles ils peuvent se lier par liaison par la force ou par la forme.

21. Fermeture pour plaies selon la revendication 19 ou 20, caractérisée en ce que les pièces de pincement (35) sont constituées par des blocs à grilles (40).

22. Fermeture pour plaies selon la revendication 21, caractérisée en ce que les blocs à grilles (40) comportent un ou plusieurs plans de grille (41) présentant des ouvertures (42) en correspondance avec les dents (39) du contre-crochet (36) de manière à assurer une liaison bloquée.

23. Fermeture pour plaies selon une des revendication 19 à 22, caractérisée en ce qu'entre le bord de l'élément de fixation (33) et l'aponévrose (1) avec laquelle il est en prise d'une part, entre cette aponévrose (1) et les pièces de pincement (35) d'autre part, sont prévus des joints d'étanchéité (37, 38) entourant totalement l'ouverture de la blessure (4).

24. Fermeture pour plaies selon une des revendication 20 à 23, caractérisée en ce que les pièces de pincement (35) sont espacées les unes des autres afin de permettre de les déplacer latéralement, le long de l'ouverture de la blessure (4).

25. Fermeture pour plaies selon la revendication 20, caractérisée en ce que les pièces de pincement (35) sont faites d'un matériau plein flexible élastiquement dans lequel peuvent pénétrer les contre-crochets (36).

26. Fermeture pour plaies selon la revendication 19, caractérisée en ce que le bord interne (34) de la portée (33a) de l'élément de fixation (33) présente en surface, tout autour de l'ouverture de la blessure (4) un évidement (43) se rétrécissant superficiellement en direction de l'aponévrose (1) en coupe de façon qu'on peut engager et bloquer après interposition de l'aponévrose (1) associée le cas échéant à d'autres couches de tissus organiques, une ou plusieurs pièces de pincement (35) jouant le rôle d'organe d'étanchéité et/ou de maintien.
